Europäisches Patentamt

European Patent Office

Office européen des brevets

(11) Veröffentlichungsnummer: **0 028 709**
B1

## (12) EUROPÄISCHE PATENTSCHRIFT

(45) Veröffentlichungstag der Patentschrift:
29.01.86

(51) Int. Cl.⁴: **C 07 C 59/21**, C 07 C 51/58

(21) Anmeldenummer: **80106117.7**

(22) Anmeldetag: **08.10.80**

(54) Verfahren zur Herstellung von gamma-Chloracetessigsäurechlorid.

(30) Priorität: **01.11.79 CH 9803/79**

(43) Veröffentlichungstag der Anmeldung:
**20.05.81 Patentblatt 81/20**

(45) Bekanntmachung des Hinweises auf die Patenterteilung:
**29.01.86 Patentblatt 86/5**

(84) Benannte Vertragsstaaten:
**AT DE FR GB IT NL SE**

(56) Entgegenhaltungen:
**US - A - 3 950 412**

**Chemical Abstracts Band 86, Nr. 19, 9 Mai 1977
Columbus, Ohio, USA K. SEKIYAMA et al.
"Gamma-Chloroacetoacetylchloride" Seite 506, Spalte
1, Abstract Nr. 139408h**

**Die Akte enthält technische Angaben, die nach dem
Eingang der Anmeldung eingereicht wurden und die
nicht in dieser Patentschrift enthalten sind.**

(73) Patentinhaber: **LONZA AG, Gampel/Wallis (CH)**

(72) Erfinder: **Gross, Max, Dipl.Chem.-Ing.,
Burgunderstrasse 5, Biberist(Solothurn) (CH)**

(74) Vertreter: **Weinhold, Peter, Dr. et al, Patentanwälte Dr. V.
Schmied-Kowarzik Dipl.-Ing. G. Dannenberg Dr. P.
Weinhold Dr. D. Gudel Dipl.-Ing. S. Schubert Dr. P. Barz
Siegfriedstrasse 8, D-8000 München 40 (DE)**

ACTORUM AG

## Beschreibung

Die Erfindung betrifft ein Verfahren zur Herstellung von γ-Chloracetessigsäurechlorid aus Chlor und Diketen, wobei man von in einem inerten Lösungsmittel gelösten Chlor und einem in einem inerten Lösungsmittel gelösten Diketen in einem Molverhältnis von Chlor zu Diketen von 0,9 bis 1,2 zu 1 ausgeht.

Es ist bekannt, aus Chlorgas und Diketen γ-Chloracetessigsäurechlorid herzustellen.

Die Reaktion verläuft exotherm und benötigt daher hohen Kühlungsaufwand. Insbesondere bei zu langsamen Reaktionsverlauf bildet sich unerwünschtes α-Chloracetessigsäurechlorid. Gemäss japanischer Patentpublikation 113824 (1976) sollen diese Nachteile dadurch vermieden werden, dass man in einem Säulenreaktionsgefäss gelöstes Diketen herabfliessen lässt und gleichzeitig im Gleich- oder Gegenstrom verdünntes Chlorgas zuführt. Das Verfahren führt aber nur zu Selektivitäten von weniger als 90%. Zudem ist der Platz- und Erstellungsaufwand für derartige Säulenreaktionsgefässe unerwünscht hoch. Die Kapazität solcher Säulenreaktionsgefässe ist auch durch die verhältnismässig kleine Austauschfläche und kleine Strömungsgeschwindigkeiten sehr gering.

Aus der US-Patentschrift 3950412 ist bekannt, γ-Halogenacetessigsäurehalogenide aus Diketen und einem Halogen herzustellen. Da das Verfahren bei sehr tiefen Temperaturen ausgeführt werden muss, um die Bildung von beispielsweise α-Chloracetessigsäure zu vermeiden, sind die Raum-Zeit-Ausbeuten gering. Es ist Aufgabe der vorliegenden Erfindung, diese genannten Nachteile zu vermeiden.

Das Verfahren wird ausgeführt, indem man das Diketen in einem Lösungsmittel löst. Zweckmässig werden als Lösungsmittel chlorierte Kohlenwasserstoffe, wie Dichlormethan, Dichloraethan, Dichlorpropan, Kohlenstofftetrachlorid oder Chloroform, vorzugsweise Methylenchlorid, angewendet, wobei die Konzentration 1 bis 15 Gew.-% Diketen im Lösungsmittel betragen kann.

In zweckmässig dem gleichen Lösungsmittel wie das Diketen wird das Chlor in einer Konzentration von 1 bis 15 Gew.-% gelöst.

Die beiden gelösten Komponenten werden gleichzeitig in ein Reaktionsrohr im Gleichstrom geleitet. Die Reaktion setzt spontan ein. Dabei wird für das Verfahren weder eine Gasphase benötigt noch bildet sich eine solche. Gegenüber bekannten Verfahren kann der benötigte Reaktionsraum dadurch klein gehalten werden und eine aufwendige Abgasaufbereitung entfällt. Das Reaktionsrohr ist entsprechend der herzustellenden Produktmenge derart dimensioniert, dass sich durch die Edukteteilströme sicher eine turbulente Strömung mit einer Reynoldschen Zahl über 2500 im Reaktionsrohr einstellt.

Bei einer speziellen Ausführungsform des beanspruchten Verfahrens kann die turbulente Strömung durch eine Reynoldsche Zahl von 5000–20 000 charakterisiert werden.

Es liegt im Rahmen der Erfindung, statt nur eines Reaktionsrohres ein Rohrbündel einzusetzen, wobei die Bildung einer turbulenten Strömung auch dabei Voraussetzung ist.

Das Reaktionsrohr wird von aussen gekühlt. Zweckmässig bietet sich eine Solekühlung an. Die Kühlung soll so dimensioniert werden, dass eine Reaktionstemperatur unter dem Siedepunkt des Lösungsmittels eingehalten werden kann.

Die gelösten Edukte ihrerseits werden mit Raumtemperatur in den Reaktor eingeführt. Im Hinblick auf die Kühlkapazität kann auch mit vorgekühlten Edukten, beispielsweise bis –30 °C, gearbeitet werden.

Die Reaktion verläuft im Reaktionsrohr nahezu quantitativ ab, wobei, bezogen auf das Reaktorvolumen, hohe Durchsätze erreicht werden.

Im erfindungsgemässen Verfahren werden Selektivitäten von über 98% erreicht. Es ist verständlich, dass bei diesen hohen Selektivitäten die Bildung von unerwünschten Nebenprodukten äusserst gering ist und Nebenprodukte, wie α,γ-Dichloracetessigsäurechlorid, nur noch in Spuren nachweisbar sind.

Das γ-Chloracetessigsäurechlorid kann aus dem Reaktionsgemisch durch destillative Trennung, insbesondere vom Lösungsmittel, gewonnen werden. Es ist aber auch möglich, das γ-Chloracetessigsäurechlorid im inerten Lösungsmittel zu belassen und das Reaktionsgemisch z.B. durch Einfliessenlassen von Alkoholen, Phenolen, Aminen oder Anilin zu den entsprechenden Estern, Amiden oder Aniliden umzusetzen. Durch destillative Abtrennung des Lösungsmittels werden die Verbindungen in reiner Form gewonnen. Das destillierte, von Begleitsubstanzen befreite Lösungsmittel kann wieder in den Reaktionsprozess zurückgeführt werden.

Beispiele:

1. Ein Reaktionsrohr von 1 m Länge mit einem hydraulischen Innendurchmesser von 2 mm wurde waagerecht montiert, und während man das Rohr von aussen mit Sole von –20 °C kühlte, wurden dem Reaktionsrohr im Gleichstrom die beiden Lösungen zugeführt. Die Diketenlösung bestand aus 84 g Diketen (1 Mol) und 1,5 Liter Methylenchlorid, die Chlorlösung aus 76 g Chlor (1,07 Mol) und 1,5 Liter Methylenchlorid. Die beiden Lösungen wurden so dosiert, dass im Rohr eine Strömungsgeschwindigkeit von 1 m/s entstand. Als höchste Temperatur im Reaktionsmedium wurden 21 °C gemessen. Beim Austritt aus dem Reaktor wurde eine Temperatur von 2 °C gemessen. Die zum Reaktor austretende Lösung wurde aufgefangen, auf –15 °C gekühlt und das γ-Chloracetessigsäurechlorid mit aequimolarer Menge Äthylalkohol umgesetzt zum γ-Chloracetessigäthylester. Im Anschluss daran wurde das überschüssige Chlor sowie das Methylenchlorid durch Abdestillieren entfernt. Die gaschromatographische Analyse des Roh-γ-Chloracetessigäthylesters ergab einen Gehalt von 98,1% (0,4% Acetessigester, 0% α-Chloracetessigester, 0,3% α,γ-Dichloracetessigester).

2. Analog Beispiel 1, jedoch nur 42 g Diketen / 1,5 Liter Methylenchlorid und 38 g Chlor / 1,5 Liter Methylenchlorid und dafür eine Strömungsgeschwindigkeit von 1,66 m/s.

Die Analyse des Roh-γ-Chloracetessigäthylesters ergab einen Gehalt von 96,8%.

3. Analog Beispiel 1, jedoch mit 168 g Diketen / 1,5 Liter Methylenchlorid und 152 g Chlor / 1,5 Liter Methylenchlorid bei einer Strömungsgeschwindigkeit von 0,5 m/s. Gehalt des Roh-γ-Chloracetessigäthylester: 89,1%.

4. Als Reaktor wurde ein Rohr von 3 m Länge und einem hydraulischen Innendurchmesser von 4 mm verwendet. Das Rohr wurde senkrecht montiert und gekühlt mit Sole von −10°C. Die Lösungen wurden wiederum im Gleichstrom am Kopf des Reaktors eingespiesen. Die Lösungen hatten die gleichen Konzentrationen wie bei Beispiel 3. Die Strömungsgeschwindigkeit betrug jedoch 0,75 m/s. Die höchste Temperatur im Reaktionsmedium betrug 45°C. Die Analyse des γ-Chloracetessigäthylesters ergab einen Gehalt von 92,8%.

5. Analog Beispiel 4, jedoch mit 84 g Diketen / 1,5 Liter Methylenchlorid und 76 g Chlor / 1,5 Liter Methylenchlorid. Strömungsgeschwindigkeit: 1 m/s. Gehalt des erhaltenen Roh-γ-Chloracetessigäthylester: 95,3%.

## Patentansprüche

1. Verfahren zur Herstellung von γ-Chloracetessigsäurechlorid aus Chlor und Diketen, wobei man von in einem inerten Lösungsmittel gelösten Chlor und einem in einem inerten Lösungsmittel gelösten Diketen in einem Molverhältnis von Chlor zu Diketen von 0,9 bis 1,2 zu 1 ausgeht, dadurch gekennzeichnet, dass man die Ausgangslösungen kontinuierlich im Gleichstrom derart in einen Rohrreaktor einführt, dass sich die beiden Lösungen sofort innig vermischen und eine turbulente Strömung mit einer Reynoldschen Zahl über 2500 entsteht.

2. Verfahren gemäss Patentanspruch 1, dadurch gekennzeichnet, dass das inerte Lösungsmittel für Chlor und Diketen dasselbe ist.

3. Verfahren gemäss Patentansprüchen 1 und 2, dadurch gekennzeichnet, dass als inertes Lösungsmittel chlorierte Kohlenwasserstoffe angewendet werden.

4. Verfahren gemäss Patentansprüchen 1 bis 3, dadurch gekennzeichnet, dass als inertes Lösungsmittel Methylenchlorid angewendet wird.

5. Verfahren gemäss Patentansprüchen 1 bis 4, dadurch gekennzeichnet, dass das Chlor als 1 bis 15 Gew.%ige Lösung angewendet wird.

6. Verfahren gemäss Patentansprüchen 1 bis 5, dadurch gekennzeichnet, dass das Diketen als 1 bis 15 Gew.-%ige Lösung angewendet wird.

7. Verfahren gemäss Patentansprüchen 1 bis 6, dadurch gekennzeichnet, dass im Reaktionsrohr eine Temperatur, die unter dem Siedepunkt des Lösungsmittels liegt, eingehalten wird.

8. Verfahren gemäss Patentansprüchen 1 bis 7, dadurch gekennzeichnet, dass bei einer turbulenten Strömung mit einer Reynoldschen Zahl von 5000 bis 20 000 gearbeitet wird.

## Claims

1. Process for the production of γ-chloroacetoacetic acid chloride from chlorine and diketene, starting from chlorine dissolved in an inert solvent and a diketene dissolved in an inert solvent in a molar ratio of chlorine to diketene of 0.9–1.2:1, characterized in that the starting solutions are continuously introduced in co-current streams into the reactor in such manner that the two solutions mix immediately and intimately resulting in a turbulent flow having a Reynold's number above 2500.

2. Process according to claim 1, characterized in that the inert solvent is the same for chlorine and diketene.

3. Process according to claims 1 and 2, characterized in that as inert solvent chlorinated hydrocarbons are used.

4. Process according to claims 1 to 3, characterized in that as inert solvent methylene chloride is used.

5. Process according to claims 1 to 4, characterized in that chlorine is used as a 1 to 15% by weight solution.

6. Process according to claims 1 to 5, characterized in that the diketene is used as a 1 to 15% by weight solution.

7. Process according to claims 1 to 6, characterized in that in the reaction tube a temperature is maintained which is below the boiling point of the solvent.

8. Process according to claims 1 to 7, characterized in that it is operated with a turbulent flow having a Reynold's number of 5000 to 20 000.

## Revendications

1. Procédé pour la préparation de chlorure d'acide γ-chloroacétoacétique à partir de chlore et de dicétène, selon lequel on part de chlore dissous dans un solvant inerte et d'un dicétène dissous dans un solvant inerte dans un rapport molaire du chlore au dicétène de 0,9 à 1,2 pour 1, caractérisé en ce que les solutions de départ sont introduites en continu, en courant de même sens, dans un tube-laboratoire, de telle sorte que les deux solutions se mélangent immédiatement intimement et qu'il en résulte un courant turbulent avec un nombre de Reynolds supérieur à 2500.

2. Procédé selon la revendication 1, caractérisé en ce que le solvant inerte est le même pour le chlore et le dicétène.

3. Procédé selon les revendications 1 et 2, caractérisé en ce que l'on utilise en tant que solvant inerte des hydrocarbures chlorés.

4. Procédé selon les revendications 1 à 3, caractérisé en ce que l'on utilise en tant que solvant inerte du chlorure de méthylène.

5. Procédé selon les revendications 1 à 4, caractérisé en ce que le chlore est utilisé sous forme de solution à 1 à 15% en poids.

5  0 028 709  6

6. Procédé selon les revendications 1 à 5, caractérisé en ce que le dicétène est utilisé sous forme de solution à 1 à 15% en poids.

7. Procédé selon les revendications 1 à 6, caractérisé en ce que l'on maintient dans le tube-laboratoire une température qui se situe au-dessous du point d'ébullition du solvant.

8. Procédé selon les revendications 1 à 7, caractérisé en ce que l'on travaille avec un courant turbulent avec un nombre de Reynolds de 5000 à 20 000.